# EUROPÄISCHE PATENTSCHRIFT

(11) **EP 1 049 432 B1**
(45) Veröffentlichungstag und Bekanntmachung des Hinweises auf die Patenterteilung: **07.04.2004**
(21) Anmeldenummer: 99906053.6
(22) Anmeldetag: 15.01.1999
(51) Int. Cl.: A61F 5/01

(54) **ARMSCHIENE**
ARM SPLINT
ATTELLE POUR LE BRAS

(30) Priorität: 20.01.1998 DE 19801951
(43) Veröffentlichungstag der Anmeldung: 08.11.2000
(73) Patentinhaber: Hassler, Andreas, 83101 Rohrdorf (DE)
(72) Erfinder: Hassler, Andreas, 83101 Rohrdorf (DE)
(74) Vertreter: Tappe, Hartmut
(86) Internationale Anmeldenummer: PCT/DE1999/000073
(87) Internationale Veröffentlichungsnummer: WO 1999/037257

(56) Entgegenhaltungen:
- EP-A- 0 597 623
- WO-A-95/23568
- WO-A-95/25489
- GB-A- 1 150 072
- US-A- 4 751 920
- US-A- 5 662 594
- US-A- 5 759 165

## Beschreibung

Die vorliegende Erfindung betrifft eine Armschiene mit einem Unterarmteil und einem Oberarmteil, die über eine Gelenkeinrichtung gelenkig miteinander verbunden sind, wobei das Unterarmteil eine Unterarmaufnahme und das Oberarmteil eine Oberarmaufnahme aufweisen.

Armschienen der vorstehend genannten Art, die über eine Gelenkeinrichtung einstellbare, unterschiedliche Relativstellungen der Unterarmaufnahme zur Oberarmaufnahme aufweisen, werden in der Regel zur Therapie von Sehnen- oder Muskelverletzungen eingesetzt und sind auch unter der fachsprachlichen Bezeichnung "Quengelschiene" bekannt.

EP-A-597623 offenbart eine Armschiene dieser Art.

Nach wie vor kann jedoch beobachtet werden, daß auch nach Verwendung der vorstehend erörterten gelenkigen Armschiene Bewegungseinschränkungen am Ellenbogengelenk auftreten.

Der vorliegenden Erfindung liegt die Aufgabe zugrunde, eine Armschiene der eingangs genannten Art dahingehend zu verbessern, daß der Ausbildung von Bewegungseinschränkungen wirksam vorgebeugt wird.

Diese Aufgabe wird durch eine Armschiene mit den Merkmalen des Anspruchs 1 gelöst.

Bei der erfindungsgemäßen Armschiene ist die Unterarmaufnahme um die Längsachse des Unterarmteils verschwenkbar ausgebildet. Hierdurch wird auch bei Sehnen- oder Muskelverletzungen im Oberarmbereich der Einsatz einer frühfunktionellen Therapie möglich, die verhindert, daß durch lang andauernde Gelenkfixierungen Bewegungseinschränkungen, beispielsweise bedingt durch ein Verkleben des Sehnenapparates im Bereich des Ellenbogengelenks auftreten. Die erfindungsgemäße Armschiene ermöglicht selbst bei fixierter Beugestellung des Ellbogengelenks eine Verdrehung des Unterarms um seine Längsachse, also eine Umwendebewegung der Hand, so daß hinsichtlich dieser Bewegungsachse eine uneingeschränkte Bewegung des Unterarms und somit eine Betätigung des an diesem Bewegungsablauf beteiligten Muskel- und Sehnenapparates möglich ist. Diese Bewegungsmöglichkeit eröffnet die Möglichkeit einer sehr frühzeitigen Reaktivierung der Sehnen- und Muskelfunktion des Unterarms, selbst bei einer fixierten Armbeugestellung.

Als besonders vorteilhaft erweist es sich, wenn die Unterarmaufnahme an einem an der Gelenkeinrichtung der Armschiene angelenkten Gelenkhebel schwenkbar angeordnet ist. Hierdurch wird insgesamt ein besonders einfacher Aufbau der Armschiene möglich, wobei sowohl zur Ausbildung der Gelenkeinrichtung im Ellbogenbereich als auch zur Ausbildung des Unterarmschwenkgelenks mechanisch einfach ausgebildete Gelenke mit jeweils einem Bewegungsfreiheitsgrad einsetzbar sind. Darüber hinaus ermöglicht der Gelenkhebel über die Wahl der Gelenkhebellänge im Einzelfall eine an die gegebene Armgeometrie angepaßte Abstandseinstellung zwischen der Unterarmaufnahme und der Gelenkeinrichtung.

Zur Erzielung einer möglichst genauen und zugleich kostengünstigen Realisierung der Verschwenkbarkeit der Unterarmaufnahme gegenüber dem Gelenkhebel erweist es sich als vorteilhaft, die Unterarmaufnahme mittels einer Gleitführung gegenüber dem Gelenkhebel verschwenkbar zu gestalten. Auch ist es möglich, die Unterarmaufnahme mittels einer Pendelführung gegenüber dem Gelenkhebel verschwenkbar zu gestalten.

Eine anatomisch besonders gut angepaßte Aufnahme des Unterarms in der Unterarmaufnahme bei gleichzeitig möglichst kostengünstig ausgebildeter Gleitführung läßt sich erzielen, wenn die Unterarmaufnahme zumindest teilweise als Zylindermantelsegment ausgebildet ist, das zur Ausbildung der Gleitführung relativ verschiebbar gegenüber einer am Gelenkhebel angeordneten Führungsschiene geführt ist.

Um bevorzugte Schwenkbereiche für eine Relativverschwenkung des Unterarms gegenüber dem Ellbogen zu ermöglichen, erweist es sich als vorteilhaft, den Schwenkwinkel der Unterarmaufnahme bezüglich eines idealerweise hinsichtlich seiner Bereichsgröße und seiner Bereichsgrenzen definierten Schwenkwinkelbereichs einstellbar zu gestalten.

Insbesondere während der Anfangszeit einer Therapie ist es vorteilhaft, wenn der Schwenkwinkel der Unterarmaufnahme eine Einrichtung zur Winkelfixierung aufweist.

Zur genauen Definition des sich für den Unterarm bei einer Verdrehung um die Unterarmlängsachse ergebenden Bewegungsablaufs kann die Unterarmaufnahme zusätzlich mit einer Einrichtung zur Fixierung der Handstellung gegenüber der Unterarmaufnahme versehen sein.

Eine in anatomischer Hinsicht besonders günstige Realisierung der Einrichtung zur Fixierung der Handstellung ist möglich, wenn diese Einrichtung durch einen an der Unterarmaufnahme angeordneten Handgriff ausgebildet ist.

Wenn darüber hinaus der Handgriff in seiner Relativstellung gegenüber der Unterarmaufnahme veränderbar ist, werden auch genau definierte Änderungen hinsichtlich des durch die Handstellung beeinflußten Bewegungsablaufs bei einer Verdrehung des Unterarms um dessen Längsachse möglich.

Eine Maximierung des durch die verschwenkbare Anordnung der Unterarmaufnahme erzielten Effekts im Sinne der eingangs erwähnten frühfunktionellen Therapie wird möglich, wenn neben der verschwenkbaren Anordnung der Unterarmaufnahme auch die das Unterarmteil mit dem Oberarmteil der Armschiene verbindende Gelenkeinrichtung nicht nur eine Verschwenkbarkeit zur Ermöglichung der Einstellung definierter Armbeugestellungen, sondern vielmehr eine Möglichkeit zur Einstellung eines Schwenkbereichs zwischen zwei definierten Schwenkbereichsanschlägen aufweist.

Insbesondere bei fortgeschrittenem Heilungsprozeß der mit der Armschiene therapierten Sehnen- oder Muskelverletzung kann es sich als vorteilhaft auf die Beschleunigung des weiteren Heilungsverlaufes auswirken, wenn im Sinne einer weiteren, der frühfunktionellen Therapie dienenden Maßnahme das Ellbogenschwenkgelenk mit einer Einrichtung zur Einstellung einer Schwenkwiderstandskraft versehen ist. Durch eine der Extension des Armes entgegenwirkende Widerstandskraft kann etwa der Heilungsprozeß nach einem Bizepsabriß beschleunigt werden. Entsprechend kann natürlich auch die durch die Verschwenkbarkeit ermöglichte Schwenkbewegung der Unterarmaufnahme um die Längsachse des Unterarms bzw. des Unterarmteils mit einer Schwenkwiderstandskraft beaufschlagt werden.

Nachfolgend werden bevorzugte Ausführungsformen der Armschiene unter Bezugnahme auf die beigefügten Zeichnungen näher erläutert. Es zeigen:
- **Fig. 1**: eine Armschiene mit einer verschwenkbaren Unterarmaufnahme in einer ersten Ausführungsform;
- **Fig. 2**: eine Armschiene mit einer verschwenkbaren Unterarmaufnahme in einer zweiten Ausführungsform.

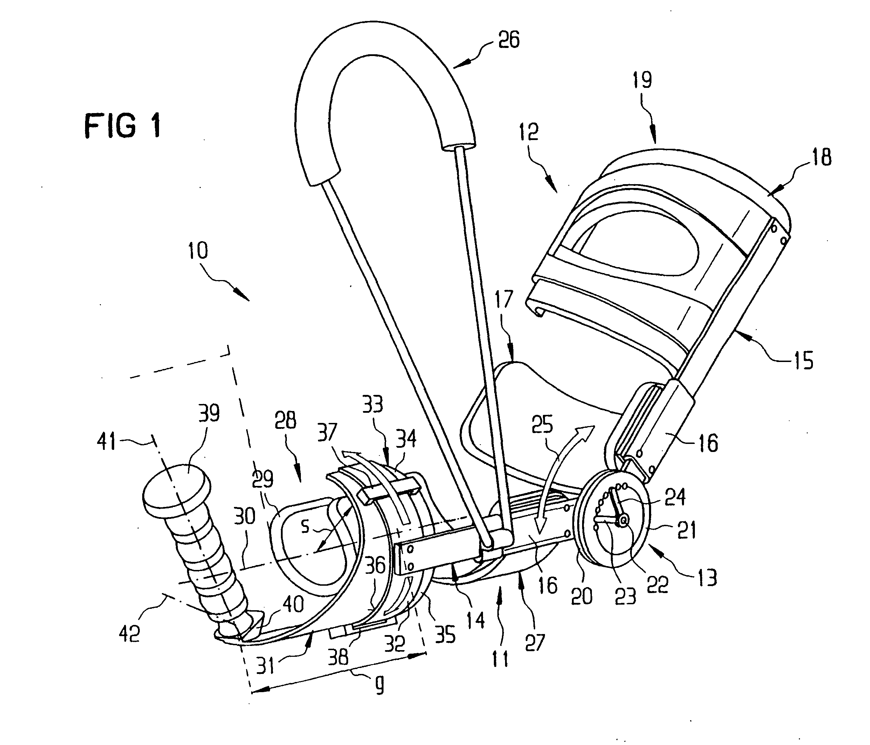

**Fig. 1** zeigt eine Armschiene 10 mit einem Unterarmteil 11 und einem Oberarmteil 12, die über ein Schwenkgelenk 13 relativ zueinander verschwenkbar miteinander verbunden sind. Sowohl das Unterarmteil 11 als auch das Oberarmteil 12 weisen in der vorliegenden Ausführungsform Gelenkhebel 14, 15 auf, die jeweils mit einer nach Art einer Teleskopverbindung ausgebildeten Längenverstelleinrichtung 16 versehen sind.

Das Oberarmteil 12 weist eine hier aus zwei gegensinnig angeordneten Halteschalen 17 und 18 gebildete Oberarmfixierungseinrichtung 19 auf, die zur kraftschlüssigen Verbindung des Oberarmteils 12 mit dem Oberarm eines Patienten dient. Die Oberarmfixierungseinrichtung 19 kann auch auf andere Art und Weise, beispielsweise durch hier nicht näher dargestellte Haltegurte, ausgebildet sein. Bei der hier dargestellten Ausführungsform mit den beiden gegensinnig zueinander angeordneten Halteschalen 17, 18 ist eine der beiden Halteschalen 17, 18 um die Längsachse des Gelenkhebels 15 verschwenkbar ausgebildet, um das Anlegen des Oberarmteils zu erleichtern.

Das Schwenkgelenk 13 weist in der hier dargestellten Ausführungsform zwei jeweils am Ende der Gelenkhebel 14 bzw. 15 angeordnete Gelenkscheiben 20, 21 auf, die über einen Schwenkbolzen 22 miteinander verbunden sind. Darüber hinaus sind an dem Schwenkgelenk 13 zwei jeweils einer Gelenkscheibe 20 bzw. 21 zugeordnete, verstellbare Schwenkanschläge 23, 24 vorgesehen, die die Definition eines Schwenkbereichs 25 ermöglichen.

Wie ferner aus der Fig. 1 hervorgeht, ist der Gelenkhebel 14 des Unterarmteils 11 mit einer Trageschlinge 26 versehen, die dazu dient, den Unterarmteil 11 in seiner Relativlage zum Rumpf des Patienten zu halten und gewichtsmäßig zu entlasten. Der Gelenkhebel 14 des im vorliegenden Fall für den linken Arm eines Patienten bestimmten Unterarmteils 11 ist neben einer relativ benachbart zum Schwenkgelenk 13 angeordneten Unterarmfixierungseinrichtung 27 mit einer Unterarmaufnahme 28 versehen, die sich bei angelegter Armschiene 10 im Bereich des hier nicht näher dargestellten Handgelenks des Patienten befindet.

Die Unterarmaufnahme 28 weist einen sich in einem Schwenkabstand s peripher zu einer Unterarmlängsachse 30 und darüber hinaus in Längsrichtung der Unterarmlängsachse 30 erstreckenden Schalenkörper 31 auf, an dem eine Aufnahmeschale 29 zur Aufnahme des Handgelenkbereichs angeordnet ist. Der Schalenkörper 31 ist mit einem Schwenksegment 35 flächig gegenüber einer starr mit dem Gelenkhebel 14 verbundenen Schwenkschiene 32 geführt und bildet mit dieser ein Unterarmschwenkgelenk 33. Zur Sicherung der flächigen Gleitführung einer Außengleitfläche 34 des Schwenksegments 35 gegenüber einer Innengleitfläche 36 der Schwenkschiene 32 sind an der Außengleitfläche 34 des Schwenksegments 35 Führungslaschen 37, 38 vorgesehen, durch die die Schwenkschiene 32 hindurchgeführt ist.

In Längsrichtung der Unterarmlängsachse 30 zum Unterarmschwenkgelenk 33 mit einem Griffabstand g versetzt befindet sich am Schalenkörper 31 angeordnet ein Handgriff 39, der im vorliegenden Fall über einen Keilsockel 40 um seine Längsachse 41 verdrehbar mit dem Schalenkörper 31 verbunden ist, derart, daß je nach Drehstellung des Handgriffs 39 unterschiedliche Winkeleinstellungen der Längsachse 41 des Handgriffs 39 gegenüber der Unterarmlängsachse 30 einstellbar sind. Somit ist es möglich, daß neben vorgegebenen Winkelstellungen der Hand des Patienten gegenüber dem Handgelenkbereich des Patienten, der in der Aufnahmeschale 29 ruht, auch derartige Winkelstellungen einstellbar sind, die eine dem Wohlbefinden des Patienten förderliche Winkelstellung ermöglichen. Abweichend von einer durch Einstellung definierbaren Winkelstellung des Handgriffs 39 gegenüber der Unterarmlängsachse 30 ist es auch möglich, den Handgriff 39 um eine Querachse 42 gegenüber dem Schalenkörper 31 verschwenkbar oder auch mittels einer hier nicht näher dargestellten Kugelgelenkeinrichtung beliebig gegenüber dem Schalenkörper verschwenkbar auszubilden, um beliebige Relativbewegungen der Hand des Patienten gegenüber dem Unterarm zuzulassen. Unabhängig vom Freiheitsgrad dieser Relativbewegungen ist es möglich, diesen Bewegungen beispielsweise durch eine entsprechend ausgebildete Gelenkverbindung zwischen dem Handgriff 39 und dem Schalenkörper 31 eine Widerstandskraft zu überlagern.

Die in **Fig. 1** dargestellte Ausführungsform des Unterarmschwenkgelenks 33 ermöglicht einen Schwenkbereich, der je nach Ausbildung und Anordnung von hier nicht näher dargestellten Schwenkanschlägen eine vollständige Umwendebewegung der Hand ermöglicht. Ausgehend von einer Ruhestellung, in der sich Elle und Speiche des Unterarms im wesentlichen in einer gemeinsamen Ebene und in paralleler Anordnung zueinander befinden, hat sich in der Praxis ein Verschwenkbereich von 30° in Richtung der Supinationsstellung und ein Verschwenkbereich von 60° in Richtung der Pronationsstellung als ausreichend erwiesen. Auch diese Verschwenkbewegung kann mit einer der Schwenkbewegung entgegenwirkenden Widerstandskraft beaufschlagt werden.

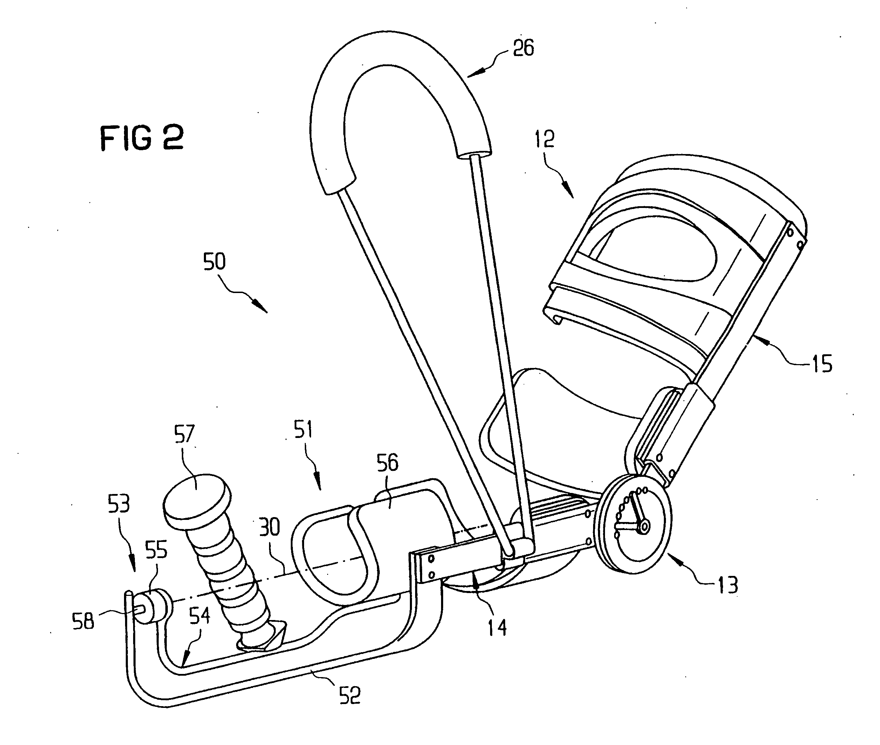

**Fig. 2** zeigt eine Armschiene 50 mit einer gegenüber der in **Fig. 1** dargestellten Armschiene 10 veränderten Ausführung eines Unterarmteils 51, wobei im übrigen entsprechend der mit **Fig. 1** übereinstimmenden Bezugszeichendefinition die Armschiene 50 dieselben Teile aufweist wie die Armschiene 10.

Die Unterarmaufnahme 51 weist einen mit dem Gelenkhebel 14 verbundenen und den Gelenkhebel 14 in Richtung der Unterarmlängsachse 30 verlängernden Ausleger 52 auf, der so geformt ist, daß ein am freien Ende des Auslegers angeordnetes Pendelgelenk 53 mit seiner Gelenkachse koaxial zur Unterarmlängsachse 30 angeordnet ist. Das Pendelgelenk 53 weist einen am freien Ende des Auslegers 52 vorgesehenen Pendelzapfen 58 und ein am freien Ende eines Pendelhebels 54 vorgesehenes Pendelauge 55 auf. An seinem entgegengesetzten Ende ist der Pendelhebel 54 mit einer Aufnahmeschale 56 versehen, die zur abstützenden Aufnahme des Handgelenkbereichs dient. Zwischen dem Pendelgelenk 53 und der Aufnahmeschale 56 ist auf dem Pendelhebel 54 angeordnet ein Handgriff 57 vorgesehen. Der Handgriff 57 kann in entsprechender Weise, wie vorstehend unter Bezugnahme auf den Handgriff 39 der in Fig. 1 dargestellten Ausführungsform der Armschiene 10 beschrieben, eine Relativbewegung gegenüber dem Pendelhebel 54 ausführen. Ebenfalls in Übereinstimmung mit der unter Bezugnahme auf Fig. 1 beschriebenen Ausbildung der Unterarmaufnahme 28 kann das Pendelgelenk 53 der Unterarmaufnahme 51 einen definierten Schwenkbereich ermöglichen.

## Patentansprüche

1. Armschiene (10) mit einem Unterarmteil (11) und einem Oberarmteil (12), die über eine Gelenkeinrichtung (13) miteinander verbunden sind, wobei das Unterarmteil (11) eine Unterarmaufnahme (28) und das Oberarmteil (12) eine Oberarmaufnahme (19) aufweisen,
**dadurch gekennzeichnet,**
**daß** die Unterarmaufnahme (28, 51) bei fixierter Beugestellung um die Längsachse (30) des Unterarmteils (11) verschwenkbar ausgebildet ist.

2. Armschiene nach Anspruch 1,
**dadurch gekennzeichnet,**
**daß** die Unterarmaufnahme (28, 51) an einem an der GeIenkeinrichtung (13) angelenkten Gelenkhebel (14) mittels eines Unterarmschwenkgelenks schwenkbar angeordnet ist.

3. Armschiene nach Anspruch 2,
**dadurch gekennzeichnet,**
**daß** zur Ausbildung des Unterarmschwenkgelenks die Unterarmaufnahme (28) mittels einer Gleitführung (33) gegenüber dem Gelenkhebel (14) verschwenkbar ist.

4. Armschiene nach Anspruch 2 oder 3,
**dadurch gekennzeichnet,**
**daß** zur Ausbildung des Unterarmschwenkgelenks die Unterarmaufnahme (51) mittels einer Pendelführung (53) gegenüber dem Gelenkhebel (14) verschwenkbar ist.

5. Armschiene nach Anspruch 3,
**dadurch gekennzeichnet,**
**daß** die Unterarmaufnahme (28) zumindest teilweise als Zylindermantelsegment (35) ausgebildet ist, das zur Ausbildung der Gleitführung (33) relativ verschiebbar gegenüber einer am Gelenkhebel (14) angeordneten Führungsschiene (32) geführt ist.

6. Armschiene nach einem oder mehreren der vorangehenden Ansprüche,
**dadurch gekennzeichnet,**
**daß** der Schwenkwinkel der Unterarmaufnahme (28, 51) bezüglich eines Schwenkwinkelbereichs (25) einstellbar ist.

7. Armschiene, nach Anspruch 6,
**dadurch gekennzeichnet,**
**daß** der Schwenkwinkel der Unterarmaufnahme (28, 51) mit einer Einrichtung zur Winkelfixierung versehen ist.

8. Armschiene nach einem oder mehreren der vorangehenden Ansprüche,
**dadurch gekennzeichnet,**
**daß** die Unterarmaufnahme (28, 51) mit einer Handstellungsfixierungseinrichtung gegenüber der Unterarmaufnahme versehen ist.

9. Armschiene nach Anspruch 8,
**dadurch gekennzeichnet,**
**daß** die Handstellungsfixierungseinrichtung durch einen an der Unterarmaufnahme (28, 51) angeordneten Handgriff (39, 57) ausgebildet ist.

10. Armschiene nach Anspruch 9,
**dadurch gekennzeichnet,**
**daß** der Handgriff (39, 57) in seiner Ralätivstellung gegenüber der Unterarmaufnahme (28, 51) veränderbar ist.

11. Armschiene nach einem oder mehreren der vorangehenden Ansprüche,
**dadurch gekennzeichnet,**
**daß** die das Unterarmteil (11) mit dem Oberarmteil (12) verbindende Gelenkeinrichtung als ein bezüglich seines Schwenkwinkelbereichs einstellbares Ellbogenschwenkgelenk (13) ausgebildet ist.

12. Armschiene nach einem oder mehreren der vorangehenden Ansprüche,
**dadurch gekennzeichnet,**
**daß** das Ellbogenschwenkgelenk (13) und/oder das Unterarmschwenkgelenk (33, 53) mit einer Einrichtung zur Einstellung einer Schwenkwiderstandskraft versehen ist.

## Revendications

1. Attelle de bras (10) avec une partie avant-bras (11) et une partie bras supérieur (12), qui sont reliées l'une à l'autre par un dispositif d'articulation (13), la partie avant-bras (11) présentant un support d'avant-bras (28) et la partie bras supérieur (12) présentant un support de bras supérieur (19),
**caractérisée en ce que**
le support d'avant-bras (28, 51) est conçu pivotable autour de l'axe longitudinal (30) de la partie avant-bras (11) en position fléchie fixe.

2. Attelle de bras suivant la revendication 1,
**caractérisée en ce que**
le support d'avant-bras (28, 51) est disposé de manière pivotable sur un des leviers articulés (14) articulés sur le dispositif d'articulation (13) au moyen d'une articulation pivotante d'avant-bras.

3. Attelle de bras suivant la revendication 2,
**caractérisée en ce que**
pour former l'articulation pivotante de l'avant-bras, le support d'avant-bras (28) peut pivoter au moyen d'un guide coulissant (33) par rapport au levier articulé (14).

4. Attelle de bras suivant la revendication 2 ou 3,
**caractérisée en ce que**
pour former l'articulation pivotante de l'avant-bras, le support d'avant-bras (51) peut pivoter au moyen d'un guide oscillant (53) par rapport au levier articulé (14).

5. Attelle de bras suivant la revendication 3,
**caractérisée en ce que**
le support d'avant-bras (28) est conçu au moins en partie sous forme d'un segment à chemise cylindrique (35), qui est guidé, pour constituer le guide coulissant (33), de manière relativement déplaçable par rapport à un rail de guidage (32) disposé sur le levier articulé (14).

6. Attelle de bras suivant l'une quelconque ou plusieurs des revendications précédentes,
**caractérisée en ce que**
l'angle de pivotement du support d'avant-bras (28, 51) est réglable au niveau d'une plage d'angle de pivotement (25).

7. Attelle de bras suivant la revendication 6,
**caractérisée en ce que**
l'angle de pivotement du support d'avant-bras (28, 51 ) est muni d'un dispositif de fixation de l'angle.

8. Attelle de bras suivant l'une quelconque ou plusieurs des revendications précédentes,
**caractérisée en ce que**
le support d'avant-bras (28, 51) est muni d'un dispositif de fixation de position de la main par rapport au support d'avant-bras.

9. Attelle de bras suivant la revendication 8,
**caractérisée en ce que**
le dispositif de fixation de position de la main est constitué par une poignée (39, 57) disposée sur le support d'avant-bras (28, 51).

10. Attelle de bras suivant la revendication 9,
**caractérisée en ce que**
la poignée (39, 57) peut changer de position relative par rapport au support d'avant-bras (28, 51).

11. Attelle de bras suivant l'une quelconque des ou plusieurs revendications précédentes,
**caractérisée en ce que**
le dispositif d'articulation reliant la partie avant-bras (11) à la partie bras supérieur (12) est conçu sous forme d'une articulation pivotante de coude (13) réglable au niveau de sa plage d'angle de pivotement.

12. Attelle de bras suivant l'une quelconque ou plusieurs des revendications précédentes,
**caractérisée en ce que**
l'articulation pivotante du coude (13) et/ou l'articulation pivotante de l'avant-bras (33, 53) est munie d'un dispositif de réglage d'une force de résistance au pivotement.

## Claims

1. An arm splint (10) with a lower arm section (11) and an upper arm section (12), which are connected to one another via a hinge device (13), the lower arm section (11) comprising a lower arm receiving element (28) and the upper arm section (12) comprising an upper arm receiving element (19), **characterised in that** the lower arm receiving element (28, 51) is constructed to be pivotable about the longitudinal axis (30) of the lower arm section (11) in a fixed bending position.

2. An arm splint according to claim 1, **characterised in that** the lower arm receiving element (28, 51) is arranged on a hinge lever (14) articulatedly connected to the hinge device (13) so as to be pivotable by means of a lower arm swivel hinge.

3. An arm splint according to claim 2, **characterised in that** the lower arm receiving element (28) is pivotable relative to the hinge lever (14) by means of a sliding guide (33) in order to form the lower arm swivel hinge.

4. An arm splint according to claim 2 or 3, **characterised in that** the lower arm receiving element (5 1 ) is pivotable relative to the hinge lever (14) by means of a pendulum guide (53) in order to form the lower arm swivel hinge.

5. An arm splint according to claim 3, **characterised in that** the lower arm receiving element (28) is at least partially constructed as a cylindrical casing segment (35), which is guided so as to be displaceable relative to a guide rail (32) arranged on the hinge lever (14) in order to form the sliding guide (33).

6. An arm splint according to one or more of the preceding claims, **characterised in that** the pivoting angle of the lower arm receiving element (28, 51) is adjustable in respect of a pivoting angle range (25).

7. An arm splint according to claim 6, **characterised in that** the pivoting angle of the lower arm receiving element (28, 51) is provided with a device for angle fixing.

8. An arm splint according to one or more of the preceding claims, **characterised in that** the lower arm receiving element (28, 51) is provided with a hand position fixing device relative to the lower arm receiving element.

9. An arm splint according to claim 8, **characterised in that** the hand position fixing device is constructed by a handle (39, 57) arranged on the lower arm receiving element (28, 51).

10. An arm splint according to claim 9, **characterised in that** the handle (39, 57) is variable in its position relative to the lower arm receiving element (28, 51).

11. An arm splint according to one or more of the preceding claims, **characterised in that** the hinge device connecting the lower arm section (11) with the upper arm section (12) is constructed as an elbow swivel hinge (13) adjustable in respect of its pivoting angle range.

12. An arm splint according to one or more of the preceding claims, **characterised in that** the elbow swivel hinge (13) and/or the lower arm swivel hinge (33, 53) is provided with a device for adjusting a pivoting resistance force.
